# EUROPEAN PATENT APPLICATION

(11) **EP 4 759 321 A1**
(43) Date of publication of application: **17.06.2026**
(21) Application number: 24852402.7
(22) Date of filing: 09.08.2024
(51) Int. Cl.: A61K 35/747, A61K 38/44, A61K 31/194, A61K 36/185, A61K 36/87, A61K 36/73, A61K 36/605, A61K 36/752, A61K 45/06, A61P 3/00

(54) **COMPOSITION FOR PREVENTING OR TREATING METABOLIC DISEASES BY USING STRAIN, THAT EXPRESSES TARTARIC ACID DEHYDRATASE, OR COMBINATION THERAPY COMPRISING SAME**

(30) Priority: 09.08.2023 KR 20230104324
(71) Applicant: GI Longevity Inc., Seongnam-si, Gyeonggi-do 13201 (KR)
(72) Inventor: JANG, Myung Ho, Seoul 05849 (KR); YANG, Bo Gie, Seoul 05849 (KR); KIM, Byung Kwon, Daejeon 34962 (KR)
(74) Representative: Vossius & Partner Patentanwälte Rechtsanwälte mbB
(86) International application number: PCT/KR2024/011890
(87) International publication number: WO 2025/034049

(57) **Abstract**

A composition for preventing or treating metabolic diseases using a strain expressing tartrate dehydratase or a combination therapy including the same is disclosed. In one aspect, when a strain of *Limosilactobacillus fermentum* expressing tartrate dehydratase is administered in combination with tartrate, citrate, or a substance including tartrate or citrate, the composition is very effective in preventing or treating metabolic diseases due to the synergistic effect of anti-obesity activity.

## Description

### Technical Field

The present disclosure relates to a composition for preventing or treating metabolic diseases using a strain expressing tartrate dehydratase or a combination therapy including the same.

### Background Art

Obesity refers to a condition in which the body has excessive fat tissue accumulated due to an increase in the size or number of fat cells, i.e., the body has more fat than it needs. Obesity is considered the leading correctable cause of death among modern people, and is a chronic disease that must be managed to lead a healthy and vibrant life. Obesity is known to be associated with various diseases, including hypertension, diabetes, insulin resistance, glucose intolerance, hyperlipidemia, angina, fatty liver, cancer, and degenerative arthritis. As metabolic diseases caused by obesity are becoming more diverse and more severe, prevention and treatment are urgently needed.

The development of obesity treatment drugs is primarily aimed at weight loss, inhibition of fat accumulation, and improvement of metabolism. Many obesity treatments currently under development and commercialized utilize a mechanism of weight loss through appetite suppression. These appetite suppressant-type obesity treatment drugs have the advantage of being highly effective in weight loss, but they have a critical problem in that their mechanism of action is to reduce food intake by acting on the central nervous system to reduce appetite, and thus their effects do not last long or cause serious side effects when used for a long period of time. Therefore, there is an urgent need to develop an obesity treatment that does not cause serious side effects.

Meanwhile, recent studies have shown that certain compounds, such as succinic acid, may play an important role in regulating metabolism and maintaining energy balance. Succinic acid is an important intermediate in the Krebs cycle, which can inhibit fat accumulation in the body by promoting efficient energy production and lipolysis (Physiological Reports. 2020; 8: e14630).

Additionally, succinic acid has been reported to activate energy expenditure in brown fat (Nature. 2018 Aug; 560(7716): 102-106.). Among the fat cells of a subject, brown fat, unlike white fat, consumes energy through heat generation, and the activity of brown fat is inversely related to age and body metabolic status, especially obesity. Succinic acid accumulation induces uncoupling protein 1 (UCP1)-dependent brown adipose tissue (BAT) thermogenesis, which prevents diet-induced obesity and improves glucose tolerance.

Succinic acid is an intermediate metabolite produced and utilized by many living organisms, excluding viruses. There are few reported cases of adverse effects due to the ingestion of succinic acid. The mechanisms of suppressing fat accumulation and activating energy consumption of brown fat are the basis for succinic acid being considered a promising candidate for the treatment of metabolic diseases such as obesity.

However, intestinal succinic acid is present at very low concentrations in germ-free mice, and its concentration has been reported to increase in the gut where microbes live (Sci Transl Med. 2014 January 22; 6(220): 220ra11.), this suggests that the accumulation of succinic acid in the intestines is due to intestinal microorganisms rather than the intake of succinic acid, which means that in order to develop treatments for metabolic diseases such as obesity, it is necessary to discover strains that can accumulate succinic acid in the intestines.

Accordingly, the present inventors discovered a strain capable of accumulating succinic acid expressing tartrate dehydratase and/or citrate lyase, and developed a composition for preventing or treating metabolic diseases using the strain.

### Disclosure of Invention

### Technical Problem

One aspect is to provide a strain expressing tartrate dehydratase.

Another aspect is to provide a strain expressing citrate lyase.

Another aspect is to provide a complex of: a strain expressing tartrate dehydratase; and tartrate or a substance including tartrate, or citrate or a substance including citrate.

Another aspect provides a pharmaceutical composition for preventing or treating a metabolic disease, including a first active substance including a strain expressing tartrate dehydratase, and a second active substance including tartrate or a substance including tartrate, or citrate or a substance including citrate.

Another aspect provides a health functional food for preventing or ameliorating metabolic diseases, including a first active substance including a strain expressing tartrate dehydratase, and a second substance including tartrate or a substance including tartrate, or citrate or a substance including citrate.

Another aspect provides use of a composition for preventing or treating metabolic diseases, the composition including: as active ingredients, a first active substance including a strain expressing tartrate dehydratase, a culture of the strain, a lysate of the strain, or a mixture thereof, and a second active substance including tartrate or a substance including tartrate, or citrate or a substance including citrate; or as an active ingredient, the first active substance wherein the first active substance is administered in combination with the second active substance.

Another aspect provides a method of ameliorating, preventing, or treating metabolic diseases, including administering, to a subject in need thereof, a composition including: as active ingredients, a first active substance including a strain expressing tartrate dehydratase or citrate lyase, a culture of the strain, a lysate of the strain, or a mixture thereof; and a second active substance including tartrate or a substance including tartrate, or citrate or a substance including citrate.

Another aspect provides a method of ameliorating, preventing, or treating metabolic diseases, including administering, to a subject in need thereof, a first active substance including a strain expressing tartrate dehydratase or citrate lyase, a culture of the strain, a lysate of the strain, or a mixture thereof; and administering a second active substance including tartrate or a substance including tartrate, or citrate or a substance including citrate.

Another aspect provides use of a composition in the manufacture of a medicament for ameliorating, preventing, or treating metabolic diseases, the composition including: as active ingredients, a first active substance including a strain expressing tartrate dehydratase, a culture of the strain, a lysate of the strain, or a mixture thereof, and a second active substance including tartrate or a substance including tartrate, or citrate or a substance including citrate; or as an active ingredient, the first active substance wherein the first active substance is administered in combination with the second active substance.

### Solution to Problem

One aspect provides a strain expressing tartrate dehydratase.

In an embodiment, the strain may further express citrate lyase or ATP-citrate lyase. In the strain, tartrate dehydratase and citrate lyase are independently expressed. The tartrate dehydratase is an enzyme that dehydrates tartrate and converts the same into succinate, and the citrate lyase is an enzyme involved in decomposing citrate and producing succinic acid.

In an embodiment, a strain expressing tartrate dehydratase and/or citrate lyase is characterized by accumulating succinate.

In an embodiment, the strain may be a Lactobacillus group strain, specifically *Limosilactobacillus fermentum.*

The Lactobacillus group is a group of microorganisms that include aerobic or facultatively anaerobic Gram-positive rod-shaped bacteria that are widely distributed in nature. Microorganisms belonging to the Lactobacillus group include *Limosilactobacillus fermentum, Lactiplantibacillus plantarum,* and *Latilactobacillus sakei.*

Lactobacillus has been reclassified into 23 taxa, including *Limosilactobacillus, Lactiplantibacillus,* and *Latilactobacillus,* and the existing strain names and the changed strain names can be used interchangeably in this specification. For example, *Limosilactobacillus fermentum* can be used interchangeably with *Lactobacillus fermentum, Lactiplantibacillus fermentum,* or *Latilactobacillus fermentum.*

The present inventors conducted research to develop a new strain and selected *Limosilactobacillus fermentum* GB102 as a candidate strain with anti-obesity activity. The strain was deposited at the Biological Resource Center of the Korea Research Institute of Bioscience and Biotechnology on September 6, 2019, under the accession number SD1335. The identical strain was deposited at the Biological Resource Center of the Korea Research Institute of Bioscience and Biotechnology on January 14, 2020, under the accession number KCTC14105BP. The strain is a probiotic strain, is harmless to the human body, and can be used without side effects.

In this specification, the term *"Limosilactobacillus fermentum* GB102"may be used interchangeably with *L. fermentum GB102 strain, Lactobacillus fermentum GB102 strain, Limosilactobacillus fermentum GB102 strain, or GB102.*

In an embodiment, the strain may be a strain having 16S rRNA consisting of the nucleotide sequence of SEQ ID NO: 1 or 16S rRNA including a nucleotide sequence having at least 98.5% nucleotide sequence identity therewith. In some embodiments, the strain may have at least 93%, 95%, 96%, 97%, 98%, 99%, 99.5%, 99.8%, 99.9% or 100% identity to the nucleotide sequence consisting of SEQ ID NO: 1 of the present specification.

In an embodiment, the strain may be a strain including a 16S rRNA gene consisting of a nucleotide sequence of SEQ ID NO:1.

In an embodiment, the strain may be a strain deposited under the accession number KCTC14105BP.

In an embodiment, the strain may be a mutant of a naturally occurring strain.

In an embodiment, the strain may be a live cell, a dead cell, or a cytoplasmic fraction obtained by disrupting a strain, and for example, a live cell.

In an embodiment, the strain may have anti-obesity activity. In some embodiments, the anti-obesity activity may include at least one activity selected from the group consisting of suppressing weight gain, increasing energy metabolism or basal metabolic rate, accumulating brown fat, activating brown fat, reducing body fat mass relative to lean body mass, suppressing hepatic steatosis, reducing blood alanine aminotransferase (AST), alanine transaminase (ALT, GPT), triglycerides, low-density cholesterol, high-density cholesterol or total cholesterol, and increasing the expression of Uncoupling protein 1 (UCP-1). Therefore, the strain may suppress weight gain.

In an embodiment, the strain may be effective in promoting comprehensive health by helping intestinal health when treating metabolic diseases such as obesity. In some embodiments, the strain may control the intestinal microflora through the production of a large amount of organic acids. When the strain is consumed in combination with prebiotics (e.g., foods including tartrate, citrate, etc.), it can promote succinic acid production while maintaining the balance of intestinal microflora, and can ameliorate bowel movement disorders felt during dietary therapy for treating metabolic diseases, thereby achieving an enhanced effect in treating metabolic diseases such as obesity.

Another aspect provides a pharmaceutical composition for preventing or treating metabolic diseases, the pharmaceutical composition including: as active ingredients, a first active substance including a strain expressing tartrate dehydratase, a culture of the strain, a lysate of the strain, or a mixture thereof, and a second active substance including tartrate or a substance including tartrate, or citrate; or as an active ingredient, the first active substance wherein the first active substance is administered in combination with the second active substance.

In an embodiment, the pharmaceutical composition for preventing or treating metabolic diseases may be a composition or complex which includes: as active ingredients, a first active substance including a strain of *Limosilactobacillus fermentum,* a culture of the strain, a lysate of the strain, or a mixture thereof, and a second active substance including tartrate or a substance including tartrate, or citrate or a substance including citrate; or as an active ingredient, the first active substance wherein the first active substance is administered in combination with the second active substance.

The "strain" is as described above.

In this specification, the term "culture" may be used interchangeably with "culture solution", "culture supernatant", "conditioned culture solution" or "conditioned medium", and may refer to the entire medium including the strain, its metabolites, surplus nutrients, and the like, obtained by culturing the strain for a certain period of time in a medium capable of supplying nutrients so that the Lactobacillus group strain can grow and survive in vitro. The culture refers to a product obtained by culturing a probiotic strain in a known medium, and the product may or may not include the strain itself. The medium may be selected from known liquid media or solid media, and may be, for example, but is not limited to, MRS liquid media, GAM liquid media, MRS agar media, GAM agar media, and BL agar media.

In this specification, the term "lysate" may be used interchangeably with "lysate" and refers to a solution or suspension in an aqueous medium of broken cells of a microorganism, such as *Limosilactobacillus fermentum.* Cell lysates include macromolecules such as DNA, RNA, proteins, peptides, carbohydrates, lipids, etc. and/or micromolecules such as amino acids, sugars, fatty acids, etc., or fractions thereof. Additionally, the lysate includes cell debris which may be smooth or granular in structure.

The culture solution may include the culture solution itself, a concentrate thereof, or a lyophilized product obtained by culturing the strain, or a culture supernatant, a concentrate thereof, or a lyophilized product obtained by removing the strain from the culture solution.

The culture solution may be obtained by culturing *Limosilactobacillus fermentum* in an appropriate medium (e.g., MRS plate medium) at a temperature of more than 10°C or less than 40°C for a certain period of time, for example, 4 to 50 hours. The culture medium and culture conditions for culturing the *Limosilactobacillus fermentum* can be appropriately selected or modified by a person of ordinary skill in the art.

In an embodiment, the first active substance may include the strain of *Limosilactobacillus fermentum* as an active ingredient alone, or may include one or more pharmaceutically acceptable carriers, excipients or diluents.

In an embodiment, the second active substance may include tartrate or a substance including the same.

The succinic acid is a type of organic acid also called tartrate, and the IUPAC name thereof is 2, 3-dihydroxybutanedioic acid, and may be represented by Formula 1:

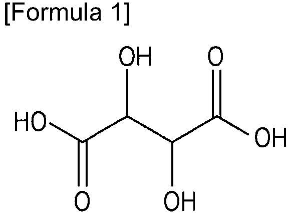

The tartrate includes L-tartrate, D-tartrate, meso-tartrate, or any combination thereof, and preferably, L-tartrate.

The tartrate may be included in the form of a salt.

The substance including the tartrate is a berry extract, for example, tamarind (*Tamarindus indica*)*,* grape (*Vitis vinifera*)*,* carambola (*Averrhoa carambola*)*,* tamarillo (*Solanum betaceum*)*,* roselle (*Hibiscus sabdariffa*)*,* citrus (*Citrus spp*.), apple (*Malus domestica*)*,* plum *(Prunus mume*)*,* date palm (*Phoenix dactylifera*)*,* cherry (*Prunus avium, Prunus cerasus*)*,* raspberry (*Rubus idaeus),* blackberry (*Rubus fruticosus*)*,* redcurrant (*Ribes rubrum*)*,* cranberry (*Vaccinium macrocarpon*), pineapple (*Ananas comosus*)*,* Japanese raisin tree (*Hovenia dulcis*)*,* banana (*Musa spp*.)*,* pomegranate (*Punica granatum*)*,* chokeberry (*Aronia melanocarpa*)*,* blueberry *(Vaccinium corymbosum*), black raspberry (*Rubus coreanus*)*,* mulberry (*Morus alba*)*,* or the like, or wine made using these berries, but is not limited thereto. In some embodiments, the substance including the tartrate is not limited in form, such as liquid or powder.

In an embodiment, the second active substance may include citrate or a substance including the same.

The citrate is a type of organic acid also called citrate, and the IUPAC name thereof is 2-hydroxypropane-1, 2, 3-tricarboxylic acid, and may be represented by Formula 2:

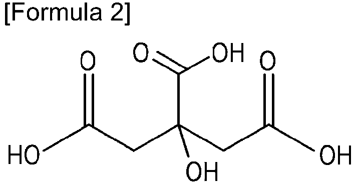

The citrate may be included in the form of a salt.

The substance including the citrate may be citrus (*Citrus spp*.) extract, specifically, extract of orange (*Citrus sinensis*)*,* lemon (*Citrus limon*)*,* lime (*Citrus aurantiifolia*)*,* grapefruit (*Citrus paradisi*), tangerine (*Citrus reticulata*)*,* or the like, but is not limited thereto. In some embodiments, the substance including the citrate is not limited in form, such as liquid or powder.

In an embodiment, the second active substance may induce succinic acid production in a strain expressing tartrate dehydratase and/or citrate lyase. That is, the second active substance may be provided as a substrate of the first active substance including a strain expressing tartrate dehydratase and/or citrate lyase.

The term "succinic acid" in this specification may be used interchangeably with succinic acid and may be represented by Formula 3:

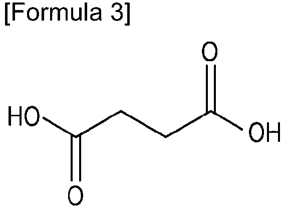

The succinic acid is administered to a subject to induce accumulation of brown fat among the subject's fat cells and activate energy consumption of brown fat. Mammals have two main types of adipose tissue: white adipose tissue and brown adipose tissue (Gesta S et al., Cell 2007; 131: 242-256). Additionally, beige adipose tissue is known to contribute to the brownization of white adipose tissue. The white adipose tissue is used for energy storage in the body and is therefore associated with obesity when in excess. In contrast, brown adipose tissue has a high concentration of mitochondria and can uniquely express uncoupling protein 1 (UCP1). Therefore, brown adipose tissue may ameliorate obesity by inducing energy consumption and thermogenesis. Therefore, succinic acid induced by the second active substance exhibits anti-obesity activity by increasing energy metabolism or basal metabolic rate through accumulating brown fat in a subject and activating energy consumption of brown fat.

In an embodiment, the first active substance and the second active substance may be administered simultaneously, sequentially, or in reverse order. For example, the anti-obesity activity of the first active substance may exhibit a synergistic effect when administered in combination with the second active substance by further inducing the ability to produce succinic acid.

The terms "combination therapy," "combined treatment," "combination administration," or "in combination" as used herein refer to any form of simultaneous or concurrent treatment using substances useful for treating at least two separate diseases. The components of the combination may be administered simultaneously, sequentially, in reverse order, or in any order. The ingredients may be administered in different dosages, at different administration frequencies, or via different routes as appropriate.

In this specification, the term "administration" refers to introducing a given substance to a subject by an appropriate method, and "subject" refers to all living things, including rats, mice, and livestock, including humans, that may have metabolic diseases. A specific example thereof could be a mammal, including humans.

The term "administered simultaneously" as used herein is not particularly limited and refers to a method in which the components of the combination therapy are administered substantially simultaneously, for example, as a mixture or in an immediately subsequent sequence.

The term "administered sequentially" as used herein is not particularly limited and refers to a method in which the components of the combination therapy are not administered simultaneously, but are administered one by one or in batches with specific time intervals between administrations. The time intervals between each administration of the components of the combination therapy may be the same or different and may be selected, for example, from 2 minutes to 96 hours, 1 day to 7 days, or 1 week, 2 weeks or 3 weeks. Typically, the time intervals between administrations may range from several minutes to several hours, for example, from 2 minutes to 72 hours, from 30 minutes to 24 hours, or from 1 to 12 hours. Additional examples include time intervals ranging from 24 to 96 hours, 12 to 36 hours, 8 to 24 hours, and 6 to 12 hours.

The combination administration may refer to administering the *Limosilactobacillus fermentum* strain and tartrate or a substance including tartrate, or citrate or a substance including citrate simultaneously, sequentially or separately, and in any order. In some embodiments, the combined administration may be simultaneously administering a first active substance including a *Limosilactobacillus fermentum* strain and a second active substance including tartrate or a substance including tartrate, or citrate or a substance including citrate, or administering the second active substance after administering the *Limosilactobacillus fermentum* strain.

A combination therapy according to the present disclosure may be defined as providing a synergistic effect where the efficacy, as measured by, for example, the degree of response, the rate of response, the time to disease progression, or the duration of survival, is therapeutically superior to the efficacy obtained by administering one or the other of the components of the combination therapy at conventional doses. For example, in the case where the therapeutic efficacy of the combination therapy is superior to that obtained by using each of the alone, the efficacy of the combination therapy is synergistic. In particular, a synergistic effect is considered to exist where the usual dose of the strain of *Limosilactobacillus fermentum* and tartrate or a substance including tartrate, or citrate or a substance including citrate, can be reduced without compromising one or more of the degree of response, the rate of response, the time to disease progression and the survival data, and in particular without compromising the duration of response, and with a reduction and/or fewer problematic side effects than would occur when each component is used at its usual dose.

The term "pharmaceutical composition" as used herein refers to a mixture including one or more active ingredients and various ingredients that deliver or assist the same for a specific therapeutic, preventive or diagnostic purpose.

The pharmaceutical composition may additionally include a pharmaceutically acceptable diluent or carrier. The diluents may be lactose, corn starch, soybean oil, microcrystalline cellulose, or mannitol, or a combination thereof. The carriers may be an excipient, a disintegrant, a binder, a glidant, or a combination thereof. The excipient may be microcrystalline cellulose, lactose, low-substituted hydroxycellulose, or a combination thereof. The disintegrant may be calcium carboxymethylcellulose, sodium starch glycolate, calcium dihydrogen phosphate anhydrous, or a combination thereof. The binder may be polyvinylpyrrolidone, low-substituted hydroxypropyl cellulose, hydroxypropyl cellulose, or a combination thereof. The lubricant may be magnesium stearate, silicon dioxide, talc, or a combination thereof.

When the pharmaceutical composition is formulated, it can be prepared using diluents or excipients such as lubricants, sweeteners, flavoring agents, emulsifiers, suspending agents, preservatives, fillers, bulking agents, binders, wetting agents, disintegrating agents, and surfactants that are commonly used. Solid preparations for oral administration may include tablets, pills, powders, granules, capsules, etc., and these solid preparations may be prepared by adding at least one excipient, such as starch, calcium carbonate, sucrose or lactose, gelatin, etc., to the composition. In addition to simple excipients, lubricants such as magnesium stearate and talc may also be used. Liquid preparations for oral administration include suspensions, solutions, emulsions, and syrups, and in addition to commonly used simple diluents such as water and liquid paraffin, various excipients, such as wetting agents, sweeteners, flavoring agents, and preservatives, may be included. Formulations for parenteral administration may include sterile aqueous solutions, non-aqueous solutions, suspensions, emulsions, lyophilized preparations, and suppositories. Non-aqueous solvents and suspending agents may includepropylene glycol, polyethylene glycol, vegetable oils such as olive oil, and injectable esters such as ethyl oleate. As a suppository base, witepsol, macrogol, tween 61, cacao butter, laurin butter, glycerogelatin, etc. can be used, and when manufacturing in the form of eye drops, known diluents or excipients may be used.

The pharmaceutical composition may include carbohydrates such as glucose, sucrose or dextran, antioxidants such as ascorbic acid or glutathione, chelating agents, low-molecular-weight proteins, or other stabilizers to increase stability or absorbability.

In an embodiment, the pharmaceutical composition may be formulated as an oral or parenteral dosage form. Oral dosage forms may be granules, powders, liquids, tablets, capsules, dry syrups, or combinations thereof. For parenteral administration, the following methods can be chosen: topical application to the skin, intraperitoneal injection, intrarectal injection, subcutaneous injection, intravenous injection, intramuscular injection, or intrathoracic injection.

In an embodiment, the route of administration of the pharmaceutical composition includes, but is not limited to, oral, intravenous, intramuscular, intraarterial, intramedullary, intrathecal, intracardiac, transdermal, subcutaneous, intraperitoneal, intranasal, enteral, topical, sublingual, or rectal routes. In some embodiments, the first active substance including a *Lactobacillus fermentum* strain and the second active substance including tartrate or a substance including tartrate, or citrate or a substance including citrate may be administered orally in the form of granules, powder, liquid, tablet, capsule, dry syrup, or a combination thereof, or may be administered parenterally by external application to the skin or by intraperitoneal injection, intrarectal injection, subcutaneous injection, intravenous injection, intramuscular injection, or intrathoracic injection.

In an embodiment, the pharmaceutical composition may include a first oral formulation including the first active substance and a second oral formulation including the second active substance, wherein the first oral formulation and the second oral formulation may be administered orally.

In an embodiment, the pharmaceutical composition may be administered orally by including the first active substance and the second active substance in a single oral formulation.

The pharmaceutical composition is administered in a pharmaceutically effective amount. The term "pharmaceutically effective amount" refers to an amount sufficient to treat a disease with a reasonable benefit/risk ratio applicable to medical treatment, and the effective dose level may be determined based on the type and severity of the patient's disease, the activity of the drug, the sensitivity to the drug, the time of administration, the route of administration and the excretion rate, the duration of treatment, concomitant medications, and other factors well known in the medical field.

According to an embodiment, the composition may include 0.001 wt% to 80 wt% of the *Limosilactobacillus fermentum* strain based on the total weight of the composition. Additionally, the dosage of the *Lactobacillus fermentum* strain may be 0.01 mg to 10, 000 mg, 0.1 mg to 1000 mg, 1 mg to 100 mg, 0.01 mg to 1000 mg, 0.01 mg to 100 mg, 0.01 mg to 10 mg, or 0.01 mg to 1 mg. The strain is included in the composition in a therapeutically effective amount or nutritionally effective concentration, and, for example, the strain is present in an amount of 10³ to 10¹⁶ CFU/g, 10³ to 10¹⁵ CFU/g, 10³ to 10¹⁴ CFU/g, 10³ to 10¹³ CFU/g, 10³ to 10¹² CFU/g, 10⁴ to 10¹⁶ CFU/g, 10⁴ to 10¹⁵ CFU/g, 10⁴ to 10¹⁴ CFU/g, 10⁴ to 10¹³ CFU/g, 10⁴ to 10¹² CFU/g, 10⁵ to 10¹⁶ CFU/g, 10⁵ to 10¹⁵ CFU/g, 10⁵ to 10¹⁴ CFU/g, 10⁵ to 10¹³ CFU/g, 10⁵ to 10¹² CFU/g, 10⁶ to 10¹³ CFU/g, 10⁶ to 10¹² CFU/g, 10⁷ to 10¹³ CFU/g, 10⁷ to 10¹² CFU/g, 10⁸ to 10¹³ CFU/g or 10⁸ to 10¹² CFU/g, or may be included in a culture of an equivalent number of live or dead cells. In some embodiments, for adult patients, 1X10³ to 1X10¹⁶ CFU/g of live or dead bacteria may be administered in one or more divided doses. However, the dosage may be prescribed in various ways depending on factors such as the formulation method, administration method, patient's age, weight, sex, pathological condition, food, administration time, administration route, excretion rate, and response sensitivity, and a person skilled in the art can appropriately adjust the dosage by taking these factors into consideration. In an embodiment, the composition includes a killed dry strain, which may be administered in an amount of 1 g to 10 g, 0.5 g to 1.5 g, 2.5 g to 3.5 g, or 4.5 g to 5.5 g, at a time.

In one aspect, the administration of the pharmaceutical composition may be administered once a day or may be administered in several divided doses. In some embodiments, it may be administered in a cycle of 6 days of administration followed by 1 day of rest, 5 days of administration followed by 2 days of rest, or 4 days of administration followed by 3 days of rest, or for example, 5 days of administration followed by 2 days of rest.

In addition, the pharmaceutically effective amount and effective dosage of the pharmaceutical composition may vary depending on the formulation method, administration method, administration time, and/or administration route of the pharmaceutical composition. In addition, it may vary depending on various factors including the type and degree of the reaction to be achieved by administering the pharmaceutical composition, the type, age, weight, general health condition, symptoms or degree of disease of the subject to be administered, sex, diet, excretion, drugs used simultaneously or concurrently in the subject, other components of the composition, and similar factors well known in the medical field. A person of ordinary skill in the art may easily determine and prescribe an effective dosage for the intended treatment. The pharmaceutical composition according to the present disclosure may be administered once a day or divided into several doses. Therefore, the dosage does not limit the scope of the present disclosure in any way. The dosage of the pharmaceutical composition may be 1 ug/kg/day to 1, 000 mg/kg/day.

The subject may be a mammal, for example, a human, cow, horse, pig, dog, sheep, goat, or cat. The subject may be a subject requiring treatment for side effects resulting from taking a metabolic disease treatment drug.

In an embodiment, the pharmaceutical composition for preventing or treating metabolic diseases may include: as active ingredients, a first active substance including a strain expressing citrate lyase, a culture of the strain, a lysate of the strain, or a mixture thereof, and a second active substance including tartrate or a substance including tartrate, or citrate or a substance including citrate; or as an active ingredient, the first active substance, wherein the first active substance is administered in combination with the second active substance.

The term "treat" as used herein may refer to curing of metabolic diseases, etc. in a shortened period of time compared to natural healing. The treatment may include amelioration and/or alleviation of metabolic diseases. Additionally, the treatment may refer to cure and/or recovery from symptoms resulting from metabolic diseases.

The term "prevention" as used herein refers to a method of partially or completely delaying or preventing the onset or recurrence of a disease, disorder, or attendant symptoms thereof, preventing the acquisition or reacquisition of a disease or disorder, or reducing the risk of acquiring a disease or disorder. For example, the prevention refers to any act of inhibiting or delaying the occurrence of metabolic diseases by administering a composition according to the present disclosure.

The term "comprising as an active ingredient" refers to the addition of a strain of *Limosilactobacillus fermentum,* a vesicle derived from the strain, a lysate of the strain, a culture solution of the strain, or an extract of the culture solution thereof, or tartrate or citrate as a main ingredient, and includes formulation in various forms by adding various ingredients as auxiliary ingredients for drug delivery, stabilization, and the like.

The term "obesity" as used herein refers to a condition in which body fat is excessive. The obesity may be clinically defined as a body mass index (BMI) of 25 or higher in Korea and 30 or higher according to the World Health Organization (WHO). Generally, obesity refers to a case where body weight is higher than normal, but obesity may exist where the proportion of body fat among body composition components is high even where body weight is not excessively high. The obesity may occur in both adults and children. The obesity may cause not only weight gain but also obesity-related metabolic diseases, such as overeating, binge drinking and bulimia, hypertension, diabetes, increased plasma insulin concentration, insulin resistance, hyperlipidemia, metabolic syndrome, insulin resistance syndrome, obesity-related gastroesophageal reflux disease, arteriosclerosis, hypercholesterolemia, hyperuricemia, cardiac hypertrophy and left ventricular hypertrophy, lipodystrophy, nonalcoholic steatohepatitis, cardiovascular disease, or polycystic ovary syndrome. Therefore, the composition may be used for the prevention or treatment of obesity as well as obesity-related metabolic diseases. In some embodiments, the composition may be used in a subject having a desire to reduce body weight or body fat even where the subject is not obese.

The obesity may be caused by various reasons. For example, the causes may include a high-fat diet, reduced exercise, genetics, psychological factors, endocrine disorders, metabolic disorders, and social and environmental factors. In particular, the obesity may be induced by a high-fat diet.

The term "metabolic disease" as used herein is a general term for various disorders that occur due to abnormalities in the body's metabolic processes, and is also called metabolic disorder. It is usually caused by an imbalance of carbohydrates, lipids, proteins, vitamins, electrolytes, and water. In some embodiments, the metabolic diseases include, but are not limited to, metabolic syndrome, type 1 diabetes, type 2 diabetes, hypertension, hyperlipidemia, obesity, fatty liver, insulin resistance, impaired glucose tolerance, angina pectoris, coronary arteriosclerosis, and arteriosclerosis.

According to an embodiment of the present disclosure, the metabolic disease may be selected from the group consisting of type 1 diabetes, type 2 diabetes, impaired glucose tolerance, impaired fasting blood glucose, dyslipidemia, lipid metabolism disorder, obesity, fatty liver, insulin resistance syndrome, and impaired glucose tolerance.

Another aspect is to provide a health functional food for preventing or ameliorating metabolic diseases, including: as active ingredients, a first active substance including a strain expressing tartrate dehydratase, a culture of the strain, a lysate of the strain, or a mixture thereof; and a second active substance including tartrate or a substance including tartrate, or citrate or a substance including citrate; or as an active ingredient, the first active substance wherein the first active substance is administered in combination with the second active substance.

With respect to the health functional food, the "strain," "first active substance," "tartrate," "citrate," "second active substance," "combination administration," and the like may be within the ranges described above.

The term "health functional food" as used herein refers to a food manufactured or processed for the purpose of health supplementation using a specific ingredient as a raw substance or by extracting, concentrating, refining, mixing, etc. a specific ingredient included in a food raw substance, and refers to a food designed and processed such that the ingredient sufficiently exerts a bioregulatory function on the body, such as biological defense, biological rhythm regulation, disease prevention, and recovery, etc. The health functional food may perform functions related to the prevention and improvement of metabolic diseases.

There are no special restrictions on the types of the foods. Examples of foods to which the extract can be added include formulations selected from the group consisting of powders, granules, tablets, capsules, pills, gels, jellies, suspensions, emulsions, syrups, tea bags, infused teas, gums, candies, and health drinks, and include all health foods in the conventional sense.

The health functional food may include food additives that are foodologically acceptable, and may include an appropriate carrier commonly used in the manufacture of health functional foods.

The term "foodologically acceptable" as used herein refers to exhibiting properties that are non-toxic to cells or humans exposed to the compound.

The health functional food may be formulated into one selected from the group consisting of tablets, pills, powders, granules, fine powders, capsules, and liquid formulations, further including one or more of a carrier, diluent, excipient, and additive. Foods to which compounds can be added according to one aspect include various foods, powders, granules, tablets, capsules, syrups, beverages, gum, tea, vitamin complexes, and health functional foods.

In addition to including the effective ingredient, the health functional food may include other ingredients as essential ingredients without special restrictions. For example, the health functional food may include additional ingredients such as various flavorings or natural carbohydrates, just like regular beverages. Examples of the natural carbohydrates described above may include common sugars such as monosaccharides, for example, glucose, fructose, etc.; disaccharides, for example, maltose, sucrose, etc.; and polysaccharides, for example, dextrin, cyclodextrin, etc., and sugar alcohols such as xylitol, sorbitol, erythritol, etc. As flavoring agents other than those described above, natural flavoring agents (thaumatin, stevia extracts (e.g., rebaudioside A, glycyrrhizin, etc.)) and synthetic flavoring agents (saccharin, aspartame, etc.) can be advantageously used. The ratio of the natural carbohydrates can be appropriately determined by a person skilled in the art.

Besides, health functional foods according to one aspect may contain various nutrients, vitamins, minerals (electrolytes), flavoring agents such as synthetic flavoring agents and natural flavoring agents, coloring agents and filling agents (cheese, chocolate, etc.), pectic acid and salts thereof, alginic acid and salts thereof, organic acids, protective colloid thickeners, pH adjusters, stabilizers, preservatives, glycerin, alcohol, carbonating agents used in carbonated beverages, and the like. These components can be used independently or in combination, and the proportions of these additives can also be appropriately selected by those skilled in the art.

In the health functional food, an active ingredient may be added directly to the food or used together with other foods or food ingredients, and may be used appropriately according to conventional methods. The amount of active ingredients mixed may be appropriately determined depending on its purpose of use (prevention or amelioration). In general, when manufacturing food or beverage, the health functional food may be added in an amount of about 15 wt% or less, for example, about 10 wt% or less, based on the raw substance. However, in case of long-term intake for health and hygiene purposes or health control purposes, the amount may be below the range.

The health functional food may be provided in combination with a health functional food for preventing or ameliorating metabolic diseases known in the past or with another existing health functional food, and the other health functional food for preventing or ameliorating metabolic diseases may be a health functional food for preventing or ameliorating metabolic diseases known in the past, an existing health functional food, or a newly developed health functional food.

Where the health functional food includes other health functional foods having a preventive or ameliorative effect on metabolic diseases, an amount capable of obtaining a maximum effect with a minimum amount without side effects may be mixed, which would be readily determined by a person skilled in the art.

Another aspect provides use of a composition for ameliorating, preventing, or treating metabolic diseases, the composition including as active ingredients: a first active substance including a strain expressing tartrate dehydratase or citrate lyase, a culture of the strain, a lysate of the strain, or a mixture thereof; and a second active substance including tartrate or a substance including tartrate, or citrate or a substance including citrate.

Another aspect is to provide a composition for ameliorating, preventing, or treating metabolic diseases, the composition including, as an active ingredient, a first active substance including a strain expressing tartrate dehydratase or citrate lyase, a culture of the strain, a lysate of the strain, or a mixture thereof, wherein the first active substance is administered in combination with tartrate or a substance including tartrate, or citrate or a substance including citrate.

Another aspect provides a method of ameliorating, preventing, or treating metabolic diseases, including administering, to a subject in need thereof, a composition including as active ingredients: a first active substance comprising a strain expressing tartrate dehydratase or citrate lyase, a culture of the strain, a lysate of the strain, or a mixture thereof; and a second active substance comprising tartrate or a substance including tartrate, or citrate or a substance including citrate.

Another aspect provides a method of ameliorating, preventing, or treating metabolic diseases, the method including: administering, to a subject in need thereof, a first active substance including a strain expressing tartrate dehydratase or citrate lyase, a culture of the strain, a lysate of the strain, or a mixture thereof; and administering a second active substance including tartrate or a substance including tartrate, or citrate or a substance including citrate.

Another aspect provides use of a composition in the manufacture of a medicament for ameliorating, preventing, or treating metabolic diseases, the composition including as active ingredients: a first active substance including a strain expressing tartrate dehydratase or citrate lyase, a culture of the strain, a lysate of the strain, or a mixture thereof; and a second active substance including tartrate or a substance including tartrate, or citrate or a substance including citrate.

Another aspect provides use of a composition in the manufacture of a medicament for ameliorating, preventing, or treating metabolic diseases, the composition including, as an active ingredient, a first active substance including a strain expressing tartrate dehydratase or citrate lyase, a culture of the strain, a lysate of the strain, or a mixture thereof, wherein the first active substance is administered in combination with tartrate or a substance including tartrate, or citrate or a substance including citrate.

The terms and methods described for the disclosure apply equally to each disclosure.

### Advantageous Effects of Invention

When a strain of *Limosilactobacillus fermentum* expressing tartrate dehydratase according to one aspect is administered in combination with tartrate, citrate, or a substance including tartrate or citrate, metabolic diseases can be effective prevented or treated due to the synergistic effect of anti-obesity activity.

### Brief Description of Drawings

FIG. 1 shows a table showing the fold changes, relative to the overall mean, in the changes in the content of intermediates in energy metabolic pathways among the metabolites present in the culture supernatants of the GB102, GB103, and *L. fermentum* strains and in the MRS culture supernatant (control).
FIG. 2 shows the distribution of genes related to dicarboxylic acid metabolism in the genomes of *Limosilactobacillus fermentum* strains.
FIG. 3 is a graph showing the results of confirming the effect of adding tartrate or citrate on the change in the content of succinic acid in the strain culture medium.
FIG. 4 is a graph measuring the body weight gain of high-fat diet mice when GB102, tartrate, or citrate were administered separately, and when GB102 and tartrate, or GB102 and citrate were administered in combination.

### Mode for the Invention

Hereinafter, preferred embodiments are presented to help understand the present disclosure. However, the following examples are provided only to make the present disclosure easier to understand, and the content of the present disclosure is not limited by the following examples. The embodiments can undergo various transformations, and the embodiments are not limited to the embodiments disclosed below and can be implemented in various forms.

### Example 1. Isolation and identification of Limosilactobacillus fermentum strains

Isolation and identification of *Limosilactobacillus fermentum* GB102 strain was performed according to the method described in Republic of Korea Patent Application No. 10-2022-0080568. The document is incorporated herein by reference in its entirety.

Briefly, the *Limosilactobacillus fermentum* strain of the present disclosure was isolated from a vaginal sample of a healthy woman who visited a hospital for the purpose of a health checkup. Specifically, vaginal internal samples were collected with a swab, streak-inoculated onto Rogosa SL (MRS) plate medium, and cultured in an anaerobic chamber at 37°C for 48 hours. As bacterial colonies grew, single colonies were subcultured onto new MRS plates for pure isolation. After pure separation, the strain was cultured using MRS medium. To identify the finally selected strain of *Limosilactobacillus fermentum* GB102, the 16S rRNA gene sequence was used for analysis. The 16S rRNA gene sequence obtained through PCR using 27F and 1492R primers targeting the 16S rRNA gene of bacteria was analyzed by Sanger sequencing, and the 16S rRNA sequence of *Limosilactobacillus fermentum* GB102 is shown in SEQ ID NO: 1. The present inventors named the GB102 strain *"Lactobacillus fermentum* GB102" (accession number: KCTC 14105BP) and deposited the same in the Korean collection for type cultures (KCTC) at the Korea Research Institute of Bioscience and Biotechnology on January 14, 2020.

Meanwhile, the strain name of *Lactobacillus fermentum* was changed to *Limosilactobacillus fermentum.* In the experimental examples below, the existing strain names and changed strain names are described interchangeably.

### Experimental Example 1. Confirmation of changes in the content of energy metabolism-related intermediates in the GB102 strain

Analysis of energy metabolism-related intermediates in MRS culture supernatant (control), GB102, GB103, and L. *fermentum* culture supernatants was performed. Specifically, changes in the content of lactic acid, pyruvic acid, fumaric acid, succinic acid, and malic acid were measured in each strain culture supernatant.

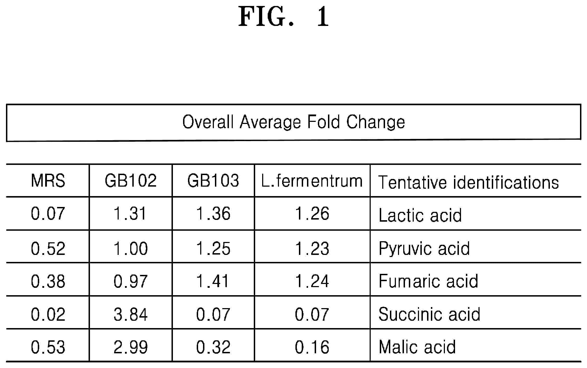

The results are shown in FIG. 1.

FIG. 1 shows a table showing the fold changes, relative to the overall mean, in the changes in the content of intermediates in energy metabolic pathways among the metabolites present in the culture supernatants of the GB102, GB103, and *L. fermentum* strains and in the MRS culture supernatant (control).

As shown in FIG. 1, succinic acid, known to be involved in the energy consumption activity of brown fat, was confirmed to be significantly increased during the culture of GB102.

This means that GB102 is effective in preventing or treating metabolic diseases such as obesity.

### Experimental Example 2. Identification of genes encoding tartrate dehydrogenase and citrate lyase in strain GB102

### 2.1. Cultivation and genome extraction of isolated strains of Lactobacillus fermentum

To determine the genomic characteristics of the GB102 strain, the genome sequence of the *Limosilactobacillus fermentum* GB102 strain was completely sequenced using next-generation sequencing technology (NGS) and bioinformatics technology.

The strain was cultured in MRS liquid medium in an anaerobic chamber at 37°C for 4 hours, and genomic DNA was extracted from the culture using a MG Genomic DNA purification kit (MGMED, Inc., Korea). To obtain long read data with an average length of 10 kb or more, sequence analysis was performed using the PacBio RS II equipment, and to produce short sequence data with a sequence length of less than 500 bp with high accuracy to supplement long read data with low accuracy, sequence analysis was performed using the NovaSeq 6000 equipment. Long read data were assembled into a high-fidelity genome draft using the HGAP2 pipeline of the SMRT Analysis server. SNP and InDel errors that may exist within the assembled genome draft sequence were corrected using long read data and short sequence data. From the complete genome sequence, coding DNA sequences (CDSs) were predicted using the Prodigal program, and rRNAs and tRNAs were predicted using the RFAM-based tool. Predicted CDSs were subjected to homology searches (using the BLAST algorithm) against publicly available databases, including the UniProt database, the GenBank nr database, the Subsystem database, the PFAM database, and the COG database, in order to predict their functions.

### 2.2. Ortholog clustering

The complete genome sequences of 34 *Lactobacillus fermentum* strains deposited in GenBank were compared with the genome sequence of strain GB102 extracted by the method of Experimental Example 2.1.

Specifically, ortholog clustering of protein-coding genes present in the genomes of strains was performed using the orthoMCL program. As a result of the clustering, it was confirmed that there were differences in the gene lists between the GB102 strain, which accumulates succinic acid, and the isolated strains that do not accumulate succinic acid. Unlike other *Lactobacillus fermentum* strains, the GB102 strain was confirmed to include both genes encoding tartrate dehydrogenase and citrate lyase, which are genes related to tricarboxylic/dicarboxylic acid metabolism.

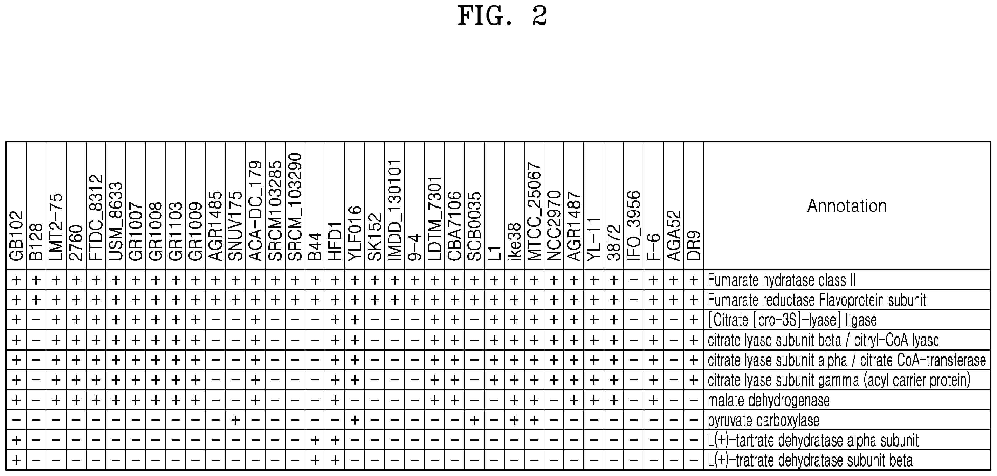

FIG. 2 shows the results of comparing the distribution of orthologs within the complete genome sequences of *Limosilactobacillus fermentum* strains deposited in GenBank and the genome sequence of the GB102 strain assembled by the method of Experimental Example 2.1, and illustrates the distribution of dicarboxylic acid metabolism-related genes in the genomes of *Limosilactobacillus fermentum* strains.

As shown in FIG. 2, among the genomes of *Limosilactobacillus fermentum* published in GenBank, only the HFD1 strain was confirmed to possess both genes encoding citrate lyase and tartrate dehydrogenase, as in the GB102 strain. Additionally, strains harboring tartrate dehydrogenase were identified as being limited to strains B44 and HFD1.

These results indicate that the presence of both genes encoding tartrate dehydrogenase and citrate lyase is not common in *Limosilactobacillus fermentum* strains.

### Experimental Example 3. Comparison of succinic acid contents in culture supernatants of strains upon addition of tartrate or citrate

The effect of addition of tartrate or citrate on changes in the succinic acid content in the culture broth of the strains was evaluated.

Specifically, 200 µL of the culture supernatant of each strain was subjected to extraction for 10 minutes in cold methanol using a mixer mill (MM400; Retsch^{®}, Haan, Germany) operated at a frequency of 30 Hz, followed by ultrasonic treatment for 10 minutes. The extracts were then centrifuged at 12,000 rpm for 10 minutes at 4°C using a refrigerated centrifuge (Centrifuge 5427R; Eppendorf, Hamburg, Germany), and the resulting supernatants were filtered through 0.2 µm PTFE (polytetrafluoroethylene) filters (Chromdisc, Daegu, Korea) and evaporated to dryness using a high-speed vacuum concentrator (Labex, Gimpo, Korea). For metabolite analysis, each dried sample was re-dissolved in 80% methanol to obtain a final concentration of 20,000 ppm. For GC-MS analysis, 100 µL of each re-dissolved sample was dried again using the high-speed vacuum concentrator and derivatized as follows.

First, 50 µL of methoxyamine hydrochloride (20 mg/mL in pyridine) was added to the dried sample, and the mixture was incubated in a heating shaker at 30°C for 90 minutes. Subsequently, 50 µL of MSTFA, used as a silylating agent, was added, and the mixture was incubated at 37°C for 30 minutes. One microliter of each derivatized sample was injected into a CHroZen GC system (Young In Chromass; Anyang, Korea) equipped with an L-PAL3 autosampler and a YL9600 mass selective detector. The metabolites were separated on a DB-5MS column (30 m length × 0.25 mm inner diameter × 0.25 µm particle size; Agilent, Santa Clara, USA) using helium as the carrier gas at a constant flow rate of 1.0 mL/min. The GC oven temperature was held at 60°C for 3 minutes, then increased at a rate of 10°C/min to 320°C, and the final temperature was held for an additional 3 minutes.

Mass data were acquired at a scan rate of 2750 amu/s over a mass range of 50-600 amu in electron ionization (EI) mode at -70 eV. The ion source and transfer line temperatures were set at 230°C and 250°C, respectively. The amounts of metabolites were expressed as peak areas in the chromatograms or as relative ratios to the mean value of all samples.

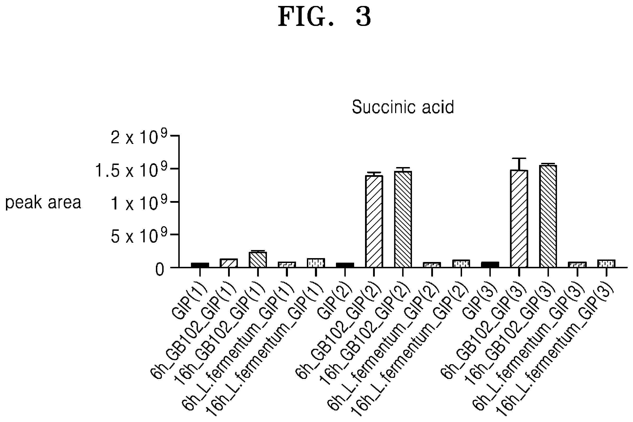

FIG. 3 shows a graph showing the results of evaluating the effect of addition of tartrate or citrate on changes in the succinic acid content in the culture broth of the strains. In FIG. 3, the culture supernatant (1) represents the succinic acid contents measured after 6 hours and 16 hours of culture of the GB102 or L. *fermentum* strain in GIP 9 medium from which citrate had been removed; culture supernatant (2) represents the succinic acid contents measured after 6 hours and 16 hours of culture of the GB102 or *L. fermentum* strain in GIP 9 medium supplemented with citrate; and culture supernatant (3) represents the succinic acid contents measured after 6 hours and 16 hours of culture of the GB102 or L. *fermentum* strain in GIP 9 medium supplemented with tartrate.

As shown in FIG. 3, L. in the culture supernatants of the L. fermentum strain, almost no change in the succinic acid content in the culture supernatant was observed depending on whether tartrate or citrate was added. In contrast, in the culture supernatants of the GB102 strain, the succinic acid content increased markedly when tartrate or citrate was added.

These results indicate that the property of increased succinic acid production upon addition of tartrate or citrate is a characteristic specific to the GB102 strain.

### Experimental Example 4. Changes in body weight gain in high-fat diet-fed mice upon co-administration of tartrate or citrate

The effect of combination administration of GB102 with tartrate or citrate on changes in body weight gain in high-fat diet-fed mice was evaluated.

Specifically, five-week-old C57BL/6 mice were acclimated for 2 weeks, after which body weights were measured and the mice were allocated into groups such that the average body weight in each group was uniform. The grouped seven-week-old mice were fed a high-fat diet (HFD) while being orally administered, once daily for 8 weeks, with the corresponding test substances for each group at a dose volume of 200 µL/head. The groups were as follows: G1: PBS; G2: GB102 (5×10⁹ CFU/head) + PBS; G3: tartrate (1 mg/head); G4: GB102 (5×10⁹ CFU/head) + tartrate (1 mg/head); G5: citrate (1 mg/head); and G6: GB102 (5×10⁹ CFU/head) + citrate (1 mg/head).

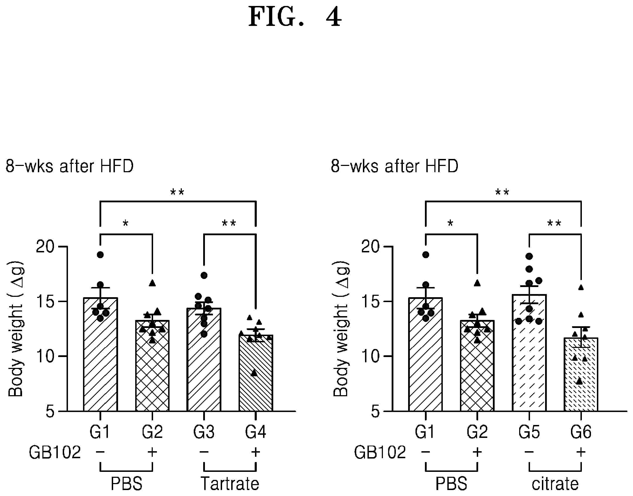

FIG. 4 shows a graph showing the body weight gain of high-fat diet-fed mice in the case where GB102, tartrate or citrate was administered alone, and in the case where GB102 was co-administered with tartrate or citrate.

As shown in FIG. 4, comparison of body weights of the respective groups after 8 weeks revealed that the group administered with GB102 exhibited a significantly (p*<0.05) lower body weight gain than the groups not administered with GB102. In particular, in the case where tartrate or citrate was co-administered with GB102, the body weight gain was significantly (p**<0.01) lower than that of the group administered with PBS.

These results indicate that administration of GB102 alone is effective in preventing or treating metabolic diseases such as obesity, and that co-administration of GB102 with tartrate or citrate is even more effective in preventing or treating metabolic diseases such as obesity.

The foregoing description of the present disclosure is for illustrative purposes, and those of ordinary skill in the art to which the present disclosure pertains will understand that the present disclosure can be easily modified into other specific forms without changing the technical spirit or essential features of the present disclosure. Therefore, the embodiments described above should be understood in all respects as illustrative and not restrictive.

### [Deposit Number]

Name of Depositary Institution: Korea Research Institute of Bioscience and Biotechnology
Accession Number: KCTC14105BP
Date of Deposit: January 14, 2020

## Claims

1. A pharmaceutical composition for preventing or treating a
metabolic disease comprising: as active ingredients,
a first active substance comprising a strain expressing tartrate dehydratase, a culture of the strain, a lysate of the strain, or a mixture thereof, and
a second active substance comprising tartrate or a substance comprising tartrate, or citrate or a substance comprising citrate; or
as an active ingredient, the first active substance, wherein the first active substance is administered in combination with the second active substance.

2. The pharmaceutical composition for preventing or treating a
metabolic disease of claim 1, wherein the strain expresses citrate lyase.

3. The pharmaceutical composition for preventing or treating a
metabolic disease of claim 1, wherein the strain is *Limosilactobacillus fermentum.*

4. The pharmaceutical composition for preventing or treating a
metabolic disease of claim 1, wherein the strain has at least 98.5% sequence identity with a 16S rRNA sequence of SEQ ID NO:1.

5. The pharmaceutical composition for preventing or treating a
metabolic disease of claim 1, wherein the strain is a strain deposited under the deposit number KCTC14105BP.

6. The pharmaceutical composition for preventing or treating a
metabolic disease of claim 1, wherein the strain comprises a naturally occurring mutant of a *Limosilactobacillus fermentum* strain.

7. The pharmaceutical composition for preventing or treating a
metabolic disease of claim 1, wherein the first active substance has anti-obesity activity.

8. The pharmaceutical composition for preventing or treating a
metabolic disease of claim 7, wherein the anti-obesity activity comprises one or more of the following:
inhibition of body weight gain;
increase in basal metabolic rate;
accumulation of brown adipose tissue;
activation of energy expenditure in brown adipose tissue;
inhibition of hepatic steatosis;
reduction of blood aspartate aminotransferase (AST), alanine transaminase (ALT, GPT), triglycerides, low-density lipoprotein cholesterol, high-density lipoprotein cholesterol, or total cholesterol; and
increase in expression of uncoupling protein 1 (UCP-1).

9. The pharmaceutical composition for preventing or treating a
metabolic disease of claim 1, wherein the tartrate is L-tartrate, D-tartrate, meso-tartrate, or any combination thereof.

10. The pharmaceutical composition for preventing or treating a
metabolic disease of claim 1, wherein the tartrate and the citrate are included in the form of a salt.

11. The pharmaceutical composition for preventing or treating a
metabolic disease of claim 1, wherein the second active substance comprises one or more extracts selected from the group consisting of tamarind (*Tamarindus indica*)*,* grape (*Vitis vinifera*)*,* carambola (*Averrhoa carambola*)*,* tamarillo (*Solanum betaceum*)*,* roselle (*Hibiscus sabdariffa*)*,* citrus (*Citrus* spp.), apple (*Malus domestica*)*,* plum (*Prunus mume*)*,* date palm (*Phoenix dactylifera*)*,* cherry (*Prunus avium, Prunus cerasus*)*,* raspberry (*Rubus idaeus*)*,* blackberry (*Rubus fruticosus*)*,* redcurrant (*Ribes rubrum*)*,* cranberry (*Vaccinium macrocarpon*)*,* pineapple (*Ananas comosus*)*,* Japanese raisin tree (*Hovenia dulcis*)*,* banana (*Musa* spp.), pomegranate (*Punica granatum*)*,* chokeberry (*Aronia melanocarpa*)*,* blueberry (*Vaccinium corymbosum*), black raspberry (*Rubus coreanus*)*,* mulberry (*Morus alba*)*.*

12. The pharmaceutical composition for preventing or treating a
metabolic disease of claim 1, wherein the metabolic disease comprises at least one selected from the group consisting of type 1 diabetes, type 2 diabetes, impaired glucose tolerance, impaired fasting blood glucose, dyslipidemia, lipid metabolism disorder, obesity, fatty liver, insulin resistance syndrome, and Impaired
glucose tolerance

13. The pharmaceutical composition for preventing or treating a
metabolic disease of claim 1, wherein the first active substance and the second active substance are administered simultaneously, sequentially or in reverse order.

14. The pharmaceutical composition for preventing or treating a
metabolic disease of claim 1, wherein the pharmaceutical composition comprises a first oral preparation comprising the first active substance and a second oral preparation comprising the second active substance, and the first oral preparation and the second oral preparation are administered orally.

15. A pharmaceutical composition for preventing or treating a
metabolic disease comprising: as active ingredients,
a first active substance comprising a strain expressing citrate lyase, a culture of the strain, a lysate of the strain, or a mixture thereof, and
a second active substance comprising tartrate or a substance comprising tartrate, citrate or a substance comprising citrate; or
as an active ingredient, the first active substance, wherein the first active substance is administered in combination with the second active substance.

16. A health functional food for preventing or ameliorating a metabolic
disease, comprising: as active ingredients,
a first active substance comprising a strain expressing tartrate dehydratase, a culture of the strain, a lysate of the strain, or a mixture thereof, and
a second active substance comprising tartrate or a substance comprising tartrate, citrate or a substance comprising citrate; or
as an active ingredient, the first active substance, wherein the first active substance is administered in combination with the second active substance.

17. Use of composition for the prevention or treatment of a metabolic
disease, the composition comprising: as active ingredients,
a first active substance comprising a strain expressing tartrate dehydratase, a culture of the strain, a lysate of the strain, or a mixture thereof, and
a second active substance comprising tartrate or a substance comprising tartrate, citrate or a substance comprising citrate; or
as an active ingredient, the first active substance, wherein the first active substance is administered in combination with the second active substance.

18. A method of ameliorating, preventing, or treating a metabolic
disease, comprising administering, to a subject in need thereof, a composition including as active ingredients: a first active substance comprising a strain expressing tartrate dehydratase or citrate lyase, a culture of the strain, a lysate of the strain, or a mixture thereof; and a second active substance comprising tartrate or a substance including tartrate, or citrate or a substance including citrate.

19. A method of ameliorating, preventing, or treating a metabolic
disease, the method comprising: administering, to a subject in need thereof, a first active substance comprising a strain expressing tartrate dehydratase or citrate lyase, a culture of the
strain, a lysate of the strain, or a mixture thereof; and administering, to a subject in need thereof a second active
substance including tartrate or a substance including tartrate, or citrate or a substance including citrate.

20. Use of a composition in the manufacture of a medicament for
ameliorating, preventing, or treating a metabolic disease, the composition comprising: as active ingredients, a first active substance including a strain expressing tartrate dehydratase, a culture of the strain, a lysate of the strain, or a mixture thereof, and a second active substance including tartrate or a substance including tartrate, or citrate or a substance including citrate; or as an active ingredient, the first active substance wherein the first active substance is administered in combination with the second active substance.
